# EUROPÄISCHE PATENTANMELDUNG

(11) **EP 2 826 851 A1**
(43) Veröffentlichungstag der Anmeldung: **21.01.2015**
(21) Anmeldenummer: 14177283.0
(22) Anmeldetag: 16.07.2014
(51) Int. Cl.: C12M 1/107, C12M 1/00, C12M 1/02

(54) **Fermenter zur Erzeugung von Biogas aus Biomasse**

(30) Priorität: 18.07.2013 DE 102013107682
(71) Anmelder: Lutz, Peter, 81925 Unterföhring (DE)
(72) Erfinder: Lutz, Peter, 81925 Unterföhring (DE)
(74) Vertreter: Alber, Norbert

(57) **Zusammenfassung**

In den Fermentern (1) nach dem Trocken-Fermentrationsverfahren einer Biogasanlage werden zumindest im Boden (1a) sowohl eine Heizvorrichtung als auch eine Perkolat-Sammeleinrichtung (4) benötigt. Zu diesem Zweck werden erfindungsgemäß Heizrohre (18) am Grund der Sammelrinne (10) angeordnet, die als Perkolat-Sammeleinrichtung (4) dient.

## Beschreibung

### I. Anwendungsgebiet

Die Erfindung betrifft einen Fermenter zur Erzeugung von Biogas aus Biomasse nach dem Trockenfermentationsverfahren.

### II. Technischer Hintergrund

Heute wird Biomasse, beispielsweise Silage oder Bio-Abfälle oder die organische Fraktion des Hausmülls, auf zwei grundsätzlich unterschiedliche Arten anaerob vergoren:
- einerseits im Nassgär-Verfahren, bei dem die Biomasse in Form einer pumpfähigen Schlempe vorliegt,
- andererseits im hier vorliegenden Feststoff-Verfahren, dem sogenannten Trockenfermentationsverfahren, bei dem die Biomasse, nur durch das Animpfen mit bereits vergorenem Material, in die Fermenter eingebracht wird.

Dabei sammelt sich das entstehende, brennbare Biogas im Freiraum oberhalb der Biomasse in jedem Fermenter und kann von dort aus abgesaugt und einem Verbraucher zugeführt werden.

Bei diesem Feststoff-Verfahren, dem so genannten Trockenfermentationsverfahren, sammelt sich Sickerflüssigkeit, das so genannte Perkolat, am Boden des Fermenters, welches aus der in der Biomasse enthaltenen Flüssigkeit besteht, die durch das Eigengewicht der Biomasse heraus gepresst wird, und die mit Fortschritt des Umsetzungsprozesses zusätzlich biologisch aktive Gär-Stoffe enthält.

Dabei ist es üblich, dieses Perkolat aus dem Bodenbereich der Fermenter abzupumpen, zwischenzuspeichern und wieder auf der Oberseite der Biomasse im Fermenter zu versprühen.

Dadurch wird das Perkolat gleichmäßig über das gesamte Volumen der Biomasse im Fermenter verteilt. Gleichzeitig kann eine Temperaturregelung im Fermenter mittels des zirkulierenden Perkolates erreicht werden, indem dieses vor der Rückführung nach Bedarf beheizt wird. Denn die Biomasse im Fermenter muss - zumindest in der Anfangsphase des Umsetzungsprozesses über längere Zeit - beheizt werden, damit die bioaktiven Mikroorganismen bei der optimalen Arbeitstemperatur arbeiten können.

Zu diesem Zweck ist es bereits bekannt, Heizrohre im Boden und ggfs. auch den Wänden des Fermenters unterzubringen, insbesondere nach Art einer Fußbodenheizung, die dort vollständig von dem Material des Fermenters umschlossen sind und somit wegen der großen Kontaktfläche gut das Material des Fermenters aufheizen. Die Heizrohre im Rahmen dieser Erfindung können auch Heizschläuche sein. Um die Wärmeverluste nach außen gering zu halten, sind die Fermenter auf der Außenseite wärmeisoliert.

Bisher wurde das Perkolat z.B. am Boden des Fermenters - der ein Gefälle zur Ladeöffnung hin aufwies - mithilfe einer quer nahe der Ladeöffnung verlaufenden Sammelrinne gesammelt. Diese Art der Entwässerung der Biomasse ist jedoch nicht optimal, da das Perkolat über die gesamte Länge des Bodens des Fermenters entlang des nur relativ geringen Gefälles zur Sammelrinne laufen muss, während die Biomasse mit hohem Druck auf den Boden drückt.

Der Nachteil dieser Lösung besteht darin, dass die Abführung von Perkolat entlang des Bodens des Fermenters nicht optimal war und das Eingießen der Heizrohre in die Fermenter bei deren Herstellung einen aufwändigen und genau durchzuführenden Arbeitsschritt darstellt.

### III. Darstellung der Erfindung

### a) Technische Aufgabe

Es ist daher die Aufgabe gemäß der Erfindung, einen Fermenter zu schaffen, der eine effizient arbeitende Perkolat-Sammeleinrichtung und eine Heizvorrichtung umfasst, die kostengünstig und einfach herzustellen sind und eine lange Lebensdauer aufweisen.

### b) Lösung der Aufgabe

Diese Aufgabe wird durch die Merkmale des Anspruchs 1 gelöst. Vorteilhafte Ausführungsformen ergeben sich aus den Unteransprüchen.

Durch die Anordnung der Heizrohre, die insbesondere im Querschnitt eine runde Außenkontur besitzen, in den Sammelrinnen, die das Perkolat über eine Abführleitung aus dem Fermenter abführen, ist das Einbringen der Heizrohre in den Fermenter mit geringerem Aufwand verbunden als bei den bisherigen Lösungen.

Zusätzlich hat das in den Sammelrinnen gesammelte Perkolat dadurch Kontakt zu den warmen Heizrohren und wird aufgeheizt, und das Perkolat gibt seinerseits diese Wärme teilweise wieder an das Material der Bodenplatte neben den Sammelrinnen ab, sodass die Bodenplatte um die Heizrohre herum stärker aufgeheizt wird als bei Lösungen, bei denen die Heizrohre sogar voll umfänglich vom Material der Bodenplatte umgeben sind.

Wenn zusätzlich die Heizrohre über mindestens die Hälfte ihres vorzugsweise in der unteren Hälfte konvex gekrümmten Außenumfanges, in Ausnahmefällen auch weniger, oder gar mehr von dem Umgebungsmaterial der Bodenplatte kontaktierend umschlossen sind, wirkt dies zusätzlich steigernd hinsichtlich des Wärmeüberganges von den Heizrohren auf die Bodenplatte.

Wenn die Sammelrinne eine größere Tiefe besitzt als der Außendurchmesser des Heizrohres, und das Heizungsrohr auf dem Boden der Sammelrinne aufliegt, ist in der Sammelrinne darüber Raum für anzusammelndes Perkolat.

Um das Verstopfen der Sammelrinne durch hineinfallende und fest gepresste Biomasse zu verhindern, ist die obere Öffnung der Sammelrinne vorzugsweise mittels einer Abdeckung abgedeckt, beispielsweise mit einem Gitter oder einem nach unten offenen U-Profil, welches auf der Oberseite Durchbrüche aufweist.

Diese Abdeckung muss sich auf dem Material der Bodenplatte abstützen, wofür ein entsprechender Vorsprung entweder auf Höhe des Gitters beidseits vorhanden sein muss, oder auf Höhe der unteren Enden des nach unten offenen U-Profiles, beispielsweise auf Höhe der Mitte des Heizrohres oder auch oberhalb der Mitte des Heizrohres.

Wenn die Heizrohre nur bis zur Mitte ihrer Höhe, also über die maximal untere Hälfte des Umfanges, in der Sammelrinne umschließend aufgenommen sind, können beschädigte Heizrohre sogar ausgetauscht werden durch Herausnahme aus der Sammelrinne nach oben.

Die Abdeckung liegt entweder mit ihrer Oberseite bündig zur Oberseite der Bodenplatte des Fermenters, oder geringfügig niedriger. Stattdessen oder zusätzlich kann die Oberseite der Abdeckung konkav gekrümmt sein, um die Belastung durch ein darüber rollendes Rad zu minimieren.

Die Sammelrinne kann einen Rinnenkörper umfassen, der die äußere Begrenzung der Sammelrinne darstellt und der auch die Schulter mit der Auflagefläche für die Abdeckung enthält, und dieser Rinnenkörper kann bei der Herstellung der Bodenplatte in diese eingegossen werden.

In Verlaufsrichtung kann sich die Sammelrinne aus mehreren hintereinander gesetzten Rinnenkörpern zusammensetzen.

Vorzugsweise verlaufen die Sammelrinnen in Längsrichtung des Fermenters und weisen insbesondere ein Gefälle zur Ladeöffnung hin auf. An mindestens einer Stelle, vorzugsweise nahe der Ladeöffnung, sind die in Längsrichtung verlaufenden Sammelrinnen durch eine quer verlaufende Verbindungsrinne miteinander verbunden, die wiederum mit der Perkolat-Sammel-einrichtung der gesamten Biogasanlage in Verbindung steht.

### c) Ausführungsbeispiele

Ausführungsformen gemäß der Erfindung sind im Folgenden beispielhaft näher beschrieben. Es zeigen:
- Fig. **1:**: eine perspektivische Ansicht eines geöffneten Fermenters nach dem Stand der Technik,
- Fig. **2**a:: eine seitliche Schnittdarstellung des Frontteiles eines geöffneten Fermenters in einer ersten Ausführungsform nach dem Stand der Technik,

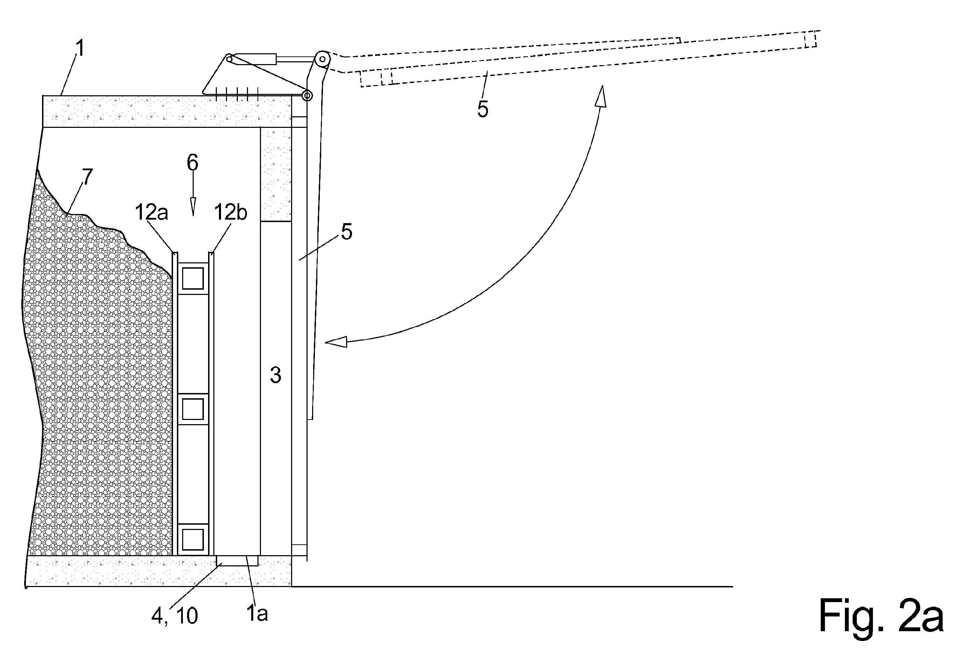

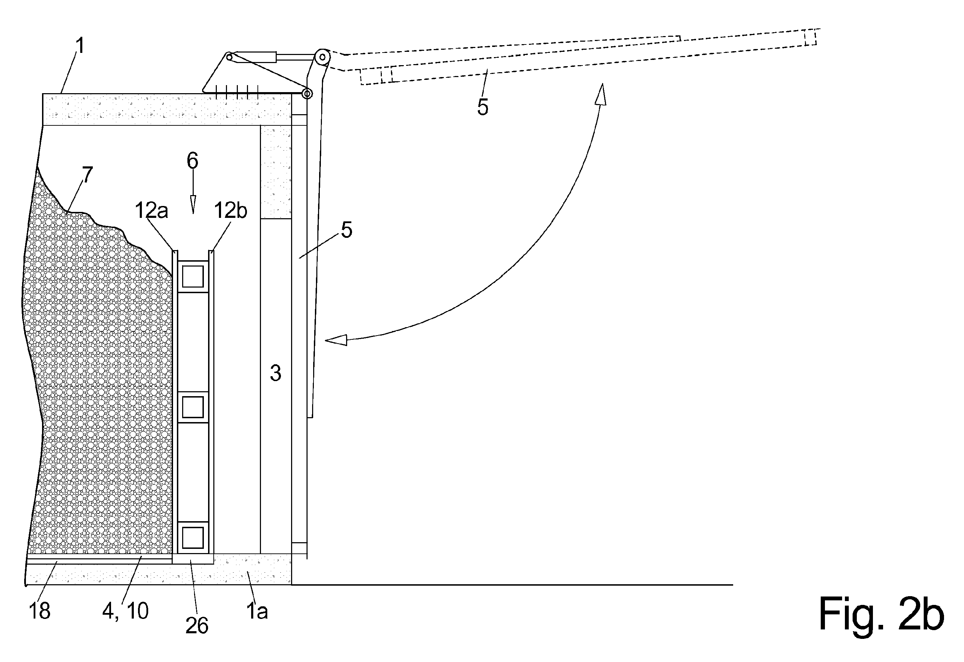

- Fig. **2**b:: eine seitliche Schnittdarstellung des Frontteiles eines geöffneten Fermenters in einer zweiten Ausführungsform gemäß der Erfindung,

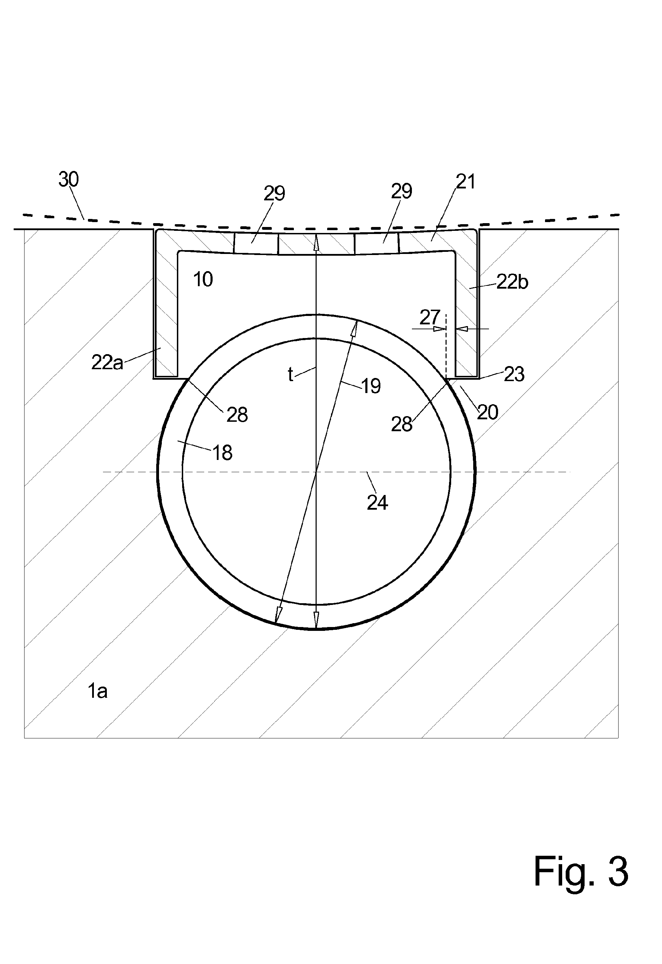

- Fig. **3:**: die erste Bauform einer Sammelrinne in Schnittdarstellung,

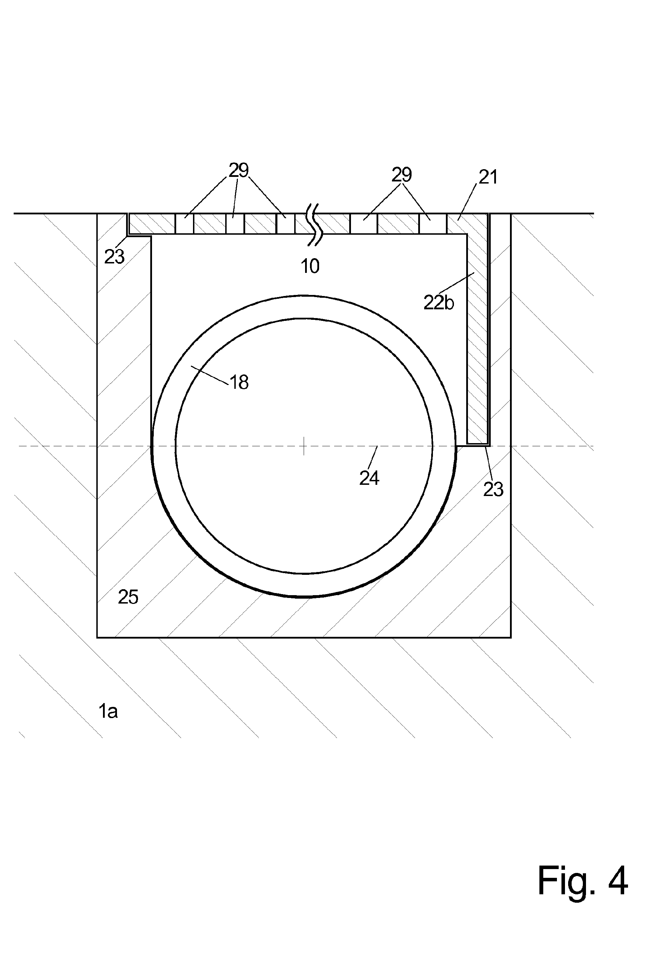

- Fig. **4:**: die zweite Bauform einer Sammelrinne in Schnittdarstellung.

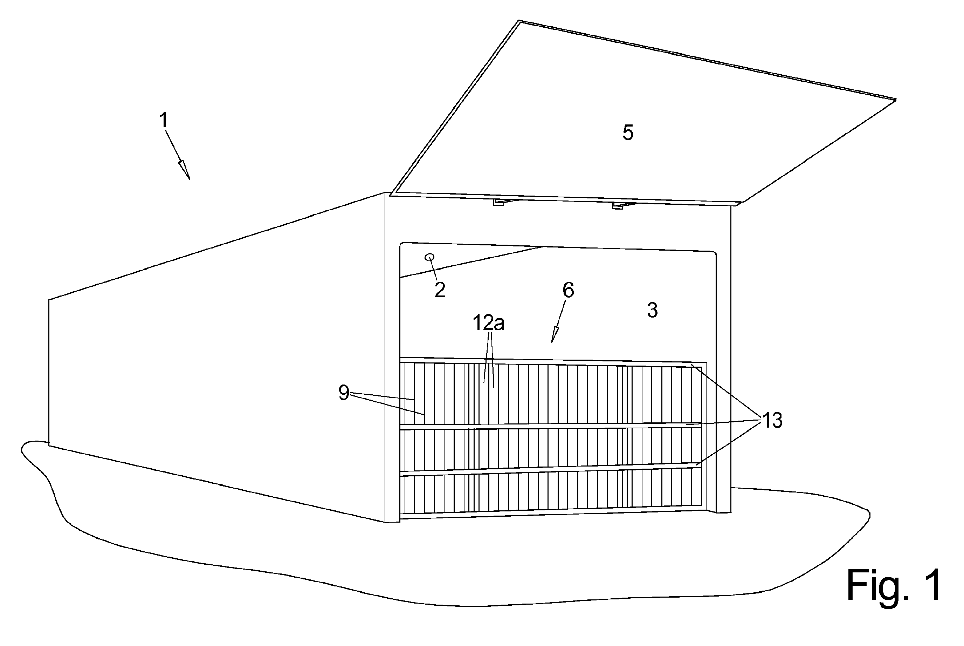

**Figur 1** zeigt einen Fermenter **1** nach dem Stand der Technik in Form eines länglichen liegenden hohlen Quaders, der in der Regel aus Beton gegossen ist und in der vorderen Stirnfläche eine großflächige Ladeöffnung **3** aufweist, die mittels der hier im geöffneten Zustand dargestellten Schließvorrichtung **5** in Form einer Klappe gasdicht und flüssigkeitsdicht verschlossen werden kann. Die größte Erstreckungsrichtung des Fermenters wird als Längsrichtung definiert. Das in dem Fermenter **1** aus der dort eingelagerten Biomasse **7** entstehende Biogas wird über die Biogas-Entnahmeöffnung **2** abgeführt.

Im Abstand geringfügig hinter der Frontfläche, also der Ladeöffnung **3,** des Fermenters **1** ist in Figur **1** die wandförmige Stützvorrichtung **6** für die dahinter aufgeschüttete Biomasse **7** montiert, die sich über die gesamte Breite der Ladeöffnung **3** erstrecken kann, also von der linken zur rechten Seitenwand des Fermenters **1** und sich vom Boden aus über etwa die Hälfte der Höhe des Fermenters **1** erstreckt.

Für das Beladen des Fermenters **1** wird diese Stützvorrichtung **6** natürlich entfernt, sodass zum Beispiel mittels eines Radladers Biomasse **7** ins Innere des Fermenters **1** gefahren und dort abgeladen werden kann, beginnend vom hinteren Ende nach vorne und so hoch aufgeschüttet wie möglich. Erst kurz vor Erreichen des vorderen Endes des Fermenters **1** mit der Füllung wird die Stützvorrichtung **6** eingebaut und zum Schluss noch Biomasse **7** über die Oberkante der Stützvorrichtung **6** in das Innere des Fermenters **1** abgekippt.

Ein Teil des Gewichts der Biomasse drückt somit gegen die nach innen weisende Rückseite der Stützvorrichtung **6,** sodass dieser Druck nicht von der Schließvorrichtung **5** aufgenommen werden muss, die dadurch sehr viel leichter gasdicht und flüssigkeitsdicht gehalten werden kann.

In Figur **1** ist die Stützvorrichtung **6** dargestellt bestehend aus einem Stützrahmen **13,** der lediglich auf der innen liegenden Rückseite mit einer Beplankung **12a** versehen ist, die aus vertikal verlaufenden Planken besteht, die mit seitlichem Abstand **9** zueinander auf dem Stützrahmen **13** montiert sind.

**Figur 2a** zeigt den Frontbereich des Fermenters **1** nach dem Stand der Technik in der Seitenansicht geschnitten und bei geschlossener Schließvorrichtung **5** und im befüllten Zustand, also auch mit eingebauter Stützvorrichtung **6.**

In diesem Fall ist die Stützvorrichtung **6** auf beiden Seiten des Stützrahmens **13** mit einer Beplankung **12a,** b versehen, wiederum jeweils mit im Abstand **9** zueinander angeordneten, vertikal verlaufenden Planken.

Unterhalb der Stützvorrichtung **6** befindet sich eine Sammelrinne **10** für Perkolat eingelassen im Boden **1**a des Fermenters **1,** die sich quer über vorzugsweise die gesamte Breite des Fermenters **1** erstreckt.

**Figur 2b** unterscheidet sich von der bekannten Lösung gemäß Figur **2a** dadurch, dass hier die nach oben offenen Sammelrinnen **10,** die in der Oberseite der meist aus Beton gegossenen Bodenplatte **1a** des Fermenters **1** eingelassen sind, in Längsrichtung des Fermenters verlaufen, vorzugsweise mehrfach nebeneinander, also von der Ladeöffnung **3** aus sich in die Tiefe des Fermenters **1** erstrecken. In Figur **2b** sind auch die in der Sammelrinne **10** liegenden Heizrohre **18** zu erkennen.

Am vorderen Ende der Sammelrinnen **10,** nämlich in diesem Fall unterhalb der Stützvorrichtung **6,** verläuft in Querrichtung eine Verbindungsrinne 26, die mit allen Sammelrinnen **10** in Verbindung steht und das Perkolat zur Perkolat-Sammeleinrichtung **4** und aus dem Fermenter 1 abführt.

Die **Figuren 3** **und** **4** zeigen zwei unterschiedliche Ausführungsformen der Sammelrinne **10, die mit der äußeren Umgebung um den Fermenter 1 in Verbindung steht zum Abführen des Perkolats** jeweils in Schnittdarstellung.

Bei **Figur 3** ist die Sammelrinne **10** als nach oben offener Freiraum in dem oberen Teil der Bodenplatte 1a ausgebildet, und im unteren Teil der Sammelrinne **10** liegt das Heizrohr **18.** Zu diesem Zweck ist die Tiefe t der Sammelrinne **10** größer als der Außendurchmesser **19** des Heizrohres **18.**

Der untere Teil der Sammelrinne **10** hat eine Kontur entsprechend der Außenkontur des Heizrohres **18,** und das Material der Bodenplatte 1a liegt kontaktierend dicht am insbesondere runden Außenumfang, dem Außendurchmesser **(19),** des Heizrohres **18** an, und zwar nicht nur in der unteren Hälfte, sondern über die horizontale Querschnittsmitte **24** nach oben hinaus, in diesem Fall über etwa **80** % der Querschnittshöhe des Heizrohres **18.**

Hierdurch wird ein in der oberen Hälfte das Heizungsrohr **18** formschlüssig haltender Haltevorsprung **20** auf jeder Seite des Querschnittes gebildet.

Dadurch ist ein guter Wärmeübergang von dem mittels eines Wärmeträgermediums durchflossenen Heizrohr **18** über das Heizrohr **18** selbst an das umgebende Material der Bodenplatte 1a gegeben.

Von der im Querschnitt am höchsten liegenden Kontaktlinie **28** des eingegossenen Heizrohres **18** zum umgebenden Material der Bodenplatte 1a aus verläuft die seitliche Flanke der Sammelrinne **1** nicht vertikal nach oben, sondern bildet zunächst eine radial nach außen springende Schulter **23** auf jeder Seite der Sammelrinne, deren unterer Schenkel von der Kontaktlinie **28** aus vorzugsweise horizontal zur Seite nach außen verläuft.

Auf diesem im wesentlichen horizontalen Schenkel der Schulter **23** sitzt ein nach unten offenes U-Profil **21** mit den freien Enden seiner frei endenden Schenkel **22a,** b auf, welches als Abdeckung der Sammelrinne **10** dient, und mit seiner Oberseite auf Höhe oder knapp unter der Oberseite der Bodenplatte **1a** liegt.

Damit wie gewünscht Perkolat in die Sammelrinne **10** sickern kann, weist das U-Profil **21** in seinem oberen, horizontalen Schenkel Durchbrüche **29** auf.

Die Erstreckung des horizontalen Schenkels der Schulter **23** ist größer als die Dicke der frei endenden Schenkel **22a,** b des U-Profiles **21.** Indem das U-Profil **21** so dimensioniert ist, dass es möglichst weit außen an den Seitenwänden der Sammelrinne **10** anliegt, entsteht von der Kontaktlinie **28** bis zur Innenseite der Schenkel **22a,** b des U-Profiles **21** ein Abstand **27,** der vorzugsweise mindestens **20** %, besser mindestens **30** % der Dicke des Schenkels **22a,** b beträgt. Dieser Abstand ist als Sicherheit notwendig, damit nicht durch Verschiebungen des U-Profiles **21** ein freies Ende des Schenkels **22a,** b in Kontakt mit dem Heizrohr **18** gerät und in dieses ein Loch drückt.

In Figur **3** ist ferner die Oberseite, also der verbindende Schenkel, des U-Profiles **21** konkav ausgebildet. Die Durchbiegung ist dabei mindestens so groß gewählt, dass ein - wie angedeutet - darüber fahrendes Rad **30** z.B. eines Radladers beim Beladen des Fermenters einen geringeren Krümmungsradius besitzt als die Oberseite der Abdeckung, in diesem Fall der Oberseite des U-Profiles **21** und dadurch das Rad **30** nur auf den Randbereichen des U-Profiles aufliegt, wo es sich über die Schenkel **22a,** b auf der Schulter **23** abstützt. Der mittlere, frei und nicht abgestützte Teil der Abdeckung, in diesem Fall des U-Profiles **21,** wird durch das Rad **30** nicht belastet.

**Figur 4** unterscheidet sich von der Bauform der Figur **3** zum einen in der linken Hälfte dadurch, dass hier als Abdeckung der Sammelrinne **10** ein horizontal liegendes Gitter **14** mit Durchbrüchen **29** verwendet wird, sodass die seitliche Schulter **23,** auf der es randseitig auf der Sammelrinne **10** aufliegt, höher liegt als bei der Verwendung eines U-Profiles **21,** wie weiterhin in der rechten Bildhälfte dargestellt.

Vor allem jedoch ist in diesem Fall die Sammelrinne **10** nicht als Ausnehmung im Material der Bodenplatte **1a** ausgebildet, sondern die Sammelrinne **10** wird begrenzt und gebildet durch einen U-förmigen, nach oben offenen Rinnenkörper **25,** der außen eine z.B. rechteckige Kontur besitzt, innen jedoch die gewünschte Konturierung mit Schulter **23** zum Auflegen der Abdeckung und einer im Schnitt kreissegmentförmigen Gestaltung des unteren Bereichs der Innenkontur der Sammelrinne **10.** In diesem Fall ist dieser untere Bereich der Kontur ein Halbkreis, der genau dem Außenumfang der unteren Hälfte des Heizrohres **18** entspricht, welches somit bis zur Mitte seiner Höhe, also bis zur Querschnittmitte **24,** kontaktierend in der Innenkontur der Sammelrinne **25** liegt.

Die Kontaktlinie **28** zwischen dem Rinnenkörper **25** und dem Heizrohr **18** liegt also genau auf Mitte der Höhe des Heizrohres **18,** also auf Höhe der Querschnittsmitte **24.**

Durch die Verwendung eines solchen Rinnenkörpers **25** können die hinsichtlich der Innenkontur exakt gestalteten Sammelrinnen **10** sehr einfach in die aus Beton zu gießende Bodenplatte **1a** eingebracht werden, indem die Rinnenkörper **25,** die sich in ihrer Verlaufsrichtung aus mehreren Teilstücken zusammensetzen können, vor dem Gießen der Bodenplatte **1** in deren Schalung eingesetzt und fixiert werden und anschließend von dem Material der Bodenplatte **1a** umgossen werden, sodass danach die abgedeckte Sammelrinne **10** wiederum vorzugsweise mit der Oberseite der Bodenplatte **1a** fluchtet.

Nach dem Aushärten der Bodenplatte **1a** können die Heizrohre **18** in Rinnenkörper **25** eingelegt und ggfs. werden und die Abdeckung aufgelegt werden.

### BEZUGSZEICHENLISTE

- 1: Fermenter
- **1a**: Bodenplatte
- **2**: Biogas-Entnahmeöffnung
- **3**: Ladeöffnung
- **4**: Perkolat-Sammeleinrichtung
- **5**: Schließvorrichtung
- **6**: Stützvorrichtung
- **7**: Biomasse
- **8**: (vert.) Abstand
- **9**: (horiz.) Abstand
- **10**: Sammelrinne
- **11**: (Breite)
- **12**a, b: Beplankung
- **13**: Stützrahmen
- **14**: Gitter
**15**
**16**
- **17**: Wand
- **18**: Heizrohr
- **19**: Außendurchmesser
- **20**: Haltevorsprung
- **21**: U-Profil
- **22**a, b: Schenkel
- **23**: Schulter
- **24**: Querschnittsmitte
- **25**: Rinnenkörper
- **26**: Verbindungsrinne
- **27**: Abstand
- **28**: Kontaktlinie
- **29**: Durchbruch
- **30**: Rad

- t: Tiefe

## Patentansprüche

1. Fermenter **(1)** zur Erzeugung von Biogas aus Biomasse **(7)** nach dem Trockenfermentationsverfahren mit einer Bodenplatte **(1a),** Wänden **(17)** und einer Decke mit
- wenigstens einer im Wesentlichen vertikal ausgerichteten Ladeöffnung **(3)** zum Befüllen und Entnehmen der Biomasse **(7),**
- einer die Ladeöffnung **(3)** in geschlossenem Zustand gas- und flüssigkeitsdicht verschließenden Schließvorrichtung **(5),**
- einer wenigstens in der Bodenplatte **(1a)** angeordneten Heizvorrichtung mit Heizrohren **(18),** die von einem Wärmeträger-Medium durchströmt werden können,
- einer Perkolat-Sammeleinrichtung **(4)** mit Sammelrinnen **(10)** im Boden (1a) des Fermenters **(1),**
**dadurch gekennzeichnet, dass**
die Heizrohre **(18)** in den Sammelrinnen **(10)** angeordnet sind.

2. Fermenter nach Anspruch **1,**
**dadurch gekennzeichnet, dass**
die Heizrohre **(18)** mindestens über die Hälfte ihres Umfanges, insbesondere die untere Hälfte ihres Umfanges, dicht anliegend von dem Material der Bodenplatte **(1a),** in der Regel Beton, kontaktierend umschlossen sind.

3. Fermenter nach einem der vorhergehenden Ansprüche,
**dadurch gekennzeichnet, dass**
die Heizrohre **(18)** über in der Summe mindestens **70** % ihres Umfanges von dem Material der Bodenplatte **(1a)** dicht anliegend umschlossen sind.

4. Fermenter nach einem der vorhergehenden Ansprüche,
**dadurch gekennzeichnet, dass**
die Sammelrinne **(10)** eine größere Tiefe (t) als der Außendurchmesser **(19)** eines Heizrohres **(18)** besitzt und das Heizrohr **(18)** auf dem Boden der Sammelrinne **(10)** aufsitzt.

5. Fermenter nach einem der vorhergehenden Ansprüche,
**dadurch gekennzeichnet, dass**
die obere Öffnung der Sammelrinne **(10)** mittels einer Abdeckung, insbesondere eines Gitters **(14)** oder eines nach unten offenen U-Profiles **(21),** ggfs. mit Durchlassöffnungen **(29)** abgedeckt ist.

6. Fermenter nach einem der vorhergehenden Ansprüche,
**dadurch gekennzeichnet, dass**
das Heizrohr **(18)** formschlüssig im Material der Bodenplatte **(1a)** gehalten ist, indem im Querschnitt des Heizrohres **(18)** betrachtet das Material der Bodenplatte **(1a)** seitlich oberhalb der Mitte des Heizrohres **(18)** am Heizrohr **(18)** anliegt und damit einen beidseitigen Haltevorsprung **(20)** bezüglich des Heizrohres bildet.

7. Fermenter nach einem der vorhergehenden Ansprüche,
**dadurch gekennzeichnet, dass**
sich die Abdeckung auf dem Material der Bodenplatte **(1a)** abstützt.

8. Fermenter nach einem der vorhergehenden Ansprüche,
**dadurch gekennzeichnet, dass**
sich das U-Profil **(21)** als Abdeckung mit den nach unten weisenden freien Enden seiner Schenkel **(22a,** b) auf den Vorsprüngen **(20)** des Materials der Bodenplatte **(1a)** abstützt, die zu diesem Zweck eine Schulter **(23)** bilden.

9. Fermenter nach einem der vorhergehenden Ansprüche,
**dadurch gekennzeichnet, dass**
die Schulter **(23)** auf oder oberhalb der Höhe der Querschnittsmitte des Heizrohres **(18)** liegt.

10. Fermenter nach einem der vorhergehenden Ansprüche,
**dadurch gekennzeichnet, dass**
die Oberseite der Abdeckung bündig oder tiefer liegt als die Oberseite der Bodenplatte **(1a),** und insbesondere die Oberseite der Abdeckung konkav gekrümmt ist.

11. Fermenter nach einem der vorhergehenden Ansprüche,
**dadurch gekennzeichnet, dass**
die Sammelrinne **(10)** einen Rinnenkörper **(25)** umfasst, der in der insbesondere aus Beton bestehenden Bodenplatte **(1**a**)** des Fermenters **(1)** eingegossen ist.

12. Fermenter nach einem der vorhergehenden Ansprüche
**dadurch gekennzeichnet, dass**
der Rinnenkörper **(25)** in seiner Haupt-Verlaufsrichtung aus mehreren Teilstücken zusammengesetzt ist.

13. Fermenter nach einem der vorhergehenden Ansprüche
**dadurch gekennzeichnet, dass**
die Sammelrinnen **(10)** in Längsrichtung des Fermenters **(1)** verlaufen und insbesondere ein Gefälle zur Ladeöffnung **(3)** hin aufweisen, wo sie insbesondere durch eine quer, also parallel vor der Ladeöffnung verlaufende Verbindungsrinne miteinander verbunden sind, die mit der Perkolat-Sammeleinrichtung **(4)** verbunden ist und das Perkolat aus dem Fermenter (1) herausleitet.
